# EUROPEAN PATENT SPECIFICATION

(11) **EP 3 773 925 B1**
(45) Date of publication and mention of the grant of the patent: **13.10.2021**
(21) Application number: 19717337.0
(22) Date of filing: 17.04.2019
(51) Int. Cl.: A61Q 11/00, A61K 8/24, A61K 8/19, A61K 8/34, A61K 8/41

(54) **ORAL CARE COMPOSITION BASED ON CALCIUM PHOSPHATE POLYIONIC CLUSTERS**
MUNDPFLEGEZUSAMMENSETZUNG ENTHALTEND KALZIUM POLYIONISCHE CLUSTERS
COMPOSITION D'HYGIÈNE BUCCALE COMPRENANT DES CLUSTERS POLYIONIQUES DE CALCIUM PHOSPHATE

(30) Priority: 21.05.2018 WO PCT/CN2018/087638; 27.06.2018 EP 18180061
(43) Date of publication of application: 17.02.2021
(73) Proprietor: Unilever Global IP Limited, Wirral, CH62 AZD (GB); Unilever IP Holdings B.V., 3013 AL Rotterdam (NL)
(72) Inventor: LI, Xiaoke, Shanghai, Changning Disitrict 200335 (CN); SHAO, Changyu, Hangzhou, Zhejiang 310058 (CN); TANG, Ruikang, Hangzhou, Zhejiang 310058 (CN); WANG, Jinfang, Shanghai, Changning Disitrict 200335 (CN); ZHANG, Meili, Shanghai, Changning Disitrict 200335 (CN); ZHOU, Huanjun, Shanghai, Changning Disitrict 200335 (CN)
(74) Representative: Tansley, Sally Elizabeth
(86) International application number: PCT/EP2019/059999
(87) International publication number: WO 2019/223940

(56) References cited:
- CN-A- 107 343 857
- US-A1- 2003 152 525
- US-A1- 2011 117 206

## Description

### Technical Field of the Invention

The present invention relates to oral care compositions such as tooth pastes, powders, gums and the like. In particular this invention relates to an oral care composition comprising calcium phosphate polyionic clusters and glycerin. The invention also relates to the use of such composition for remineralizing teeth of an individual.

### Background of the Invention

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discolouring substances are often able to permeate the enamel layer. This problem occurs gradually over many years, but imparts a noticeable discoloration of the enamel of one's teeth.

In the mouth, there is a natural equilibrium between HAP being dissolved from the enamel of teeth which is referred to as demineralization, and hydroxyapatite being formed on or in the teeth from substances occurring naturally in the saliva which is referred to as remineralization. This equilibrium is shifting continuously. As long as the rate of demineralization and the rate of remineralization remain in balance, teeth remain strong and healthy.

Ca₁₀(PO₄)₆(OH)_{2(Solid)} + 8H⁺_{(aq)} ↔ 10Ca²⁺_{(aq)} + 6HPO₄²⁻_{(aq)} + 2H₂O

Exposure of the dental hard tissues to acid causes demineralization, resulting in surface softening and a decrease in mineral density. Imbalance in this process results in the formation of dental caries which occurs when more minerals are lost from teeth than can be replaced.

Efforts have been made over the years to address the problem of dissolution or demineralization of tooth enamel and the resultant formation of dental caries. Products that address tooth decay have, nevertheless, been developed, including products that employ calcium salts for remineralization of teeth.

There is continuing need for building stronger and healthier teeth. The present inventors have now found unexpectedly that the tooth remineralization efficacy can be enhanced by using calcium phosphate polyionic clusters in combination with glycerin. It has been found that such an oral care composition is effective in promoting remineralization through rapid generation of a new remineralization layer that binds to tooth surfaces to counteract enamel demineralization caused by acid erosion.

### Additional Information

CN 107343857 A discloses hydroxyapatite with an enamel-like structure and a method for preparing of the hydroxyapatite. A calcium phosphate polyionic cluster is taken as a precursor, and the hydroxyapatite with the enamel-like structure is prepared on the enamel surfaces through the precursor.

The additional information above does not describe an oral care composition comprising a combination of calcium phosphate polyionic clusters and glycerin, and especially such an oral care composition can provide enhanced tooth remineralization efficacy.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Substantially Free

"Substantially free of" for the purpose of the present invention means less than 3.0%, and preferably less than 2.0%, and more preferably less than 1.0% and most preferably less than 0.5% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 3.0%, and preferably less than 2.0%, and more preferably less than 1.0% and most preferably less than 0.5% by total weight of the oral care composition.

### Viscosity

Viscosity of a tooth paste is the value taken at room temperature (25 °C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) calcium phosphate polyionic clusters;
b) glycerin;
wherein the calcium phosphate polyionic clusters are obtained by a process comprising the steps of:
i) dissolving a water soluble calcium source in ethanol to produce an ethanol solution A having a calcium ion concentration from 5 to 60 mmol/L;
ii) dissolving phosphoric acid and triethylamine in ethanol to produce an ethanol solution B having a phosphoric acid concentration from 10 to 120 mmol/L and a triethylamine concentration from 1000 to 3900 mmol/L;
iii) mixing the solution A and the solution B and standing the mixture to obtain a calcium phosphate polyionic cluster solution;
iv) centrifuging the calcium phosphate polyionic cluster solution to obtain the calcium phosphate polyionic clusters;
wherein the water soluble calcium source is a calcium source that dissolves in water to give a concentration of at least 0.1 moles per litre at 25°C and atmospheric pressure.

In a second aspect, the present invention is directed to a composition of any of the first aspect for use in a method for remineralizing the teeth of an individual comprising the step of applying said oral care composition to at least one surface of the teeth of the individual.

All other aspects of the present invention will more readily become apparent upon considering the detailed description and examples which follow.

### Detailed Description

The calcium phosphate polyionic cluster, as used herein, refers to a cluster composed of calcium and phosphate ions which has the properties of a gel-like substance and has a certain degree of fluidity. The calcium phosphate polyionic clusters are similar to polymers but they are formed by ionic bonding and are completely composed of ions.

Their microstructure is shown in formula (I)

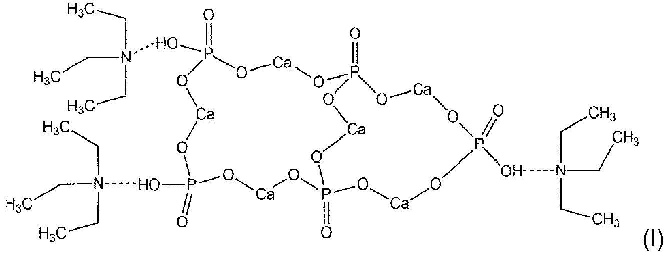

The calcium phosphate polyionic clusters suitable for use in this invention are obtained by a process comprising the steps of:
i) dissolving a water soluble calcium source in ethanol to produce an ethanol solution A having a calcium ion concentration from 5 to 60 mmol/L;
ii) dissolving phosphoric acid and triethylamine in ethanol to produce an ethanol solution B having a phosphoric acid concentration from 10 to 120 mmol/L and a triethylamine concentration from 1000 to 3900 mmol/L;
iii) mixing the solution A and the solution B and standing the mixture to obtain a calcium phosphate polyionic cluster solution;
iv) centrifuging the calcium phosphate polyionic cluster solution to obtain the calcium phosphate polyionic clusters.

The water soluble calcium source provides the calcium ion for the calcium phosphate polyionic clusters. Illustrative yet non-limiting examples of the types of the water soluble calcium source that may be used in this invention include, for example, calcium chloride, calcium nitrate, calcium acetate, calcium lactate, calcium formate, calcium malate, calcium propionate, calcium butyrate, calcium bicarbonate, calcium glycerophosphate, calcium gluconate, calcium ascorbate or mixtures thereof or the like. In a preferred embodiment, the calcium source is calcium chloride. The ethanol solution A preferably has a calcium ion concentration from 10 to 50 mmol/L, more preferably from 15 to 40 mmol/L. If the concentration of calcium ion is too low, it will reduce the yield and waste the ethanol solvent. If the concentration of calcium is too high, it will rapidly form the polyionic clusters, resulting in non-uniform reaction.

The phosphoric acid provides the phosphate anion for the calcium phosphate polyionic clusters. The ethanol solution B preferably has a phosphoric acid concentration from 20 to 100 mmol/L, more preferably from 30 to 80 mmol/L. The triethylamine absorbs some of the protons in the phosphoric acid as a base and it also behaves as a capping agent for the calcium phosphate polyionic clusters formed. The ethanol solution B preferably has a triethylamine concentration from 1100 to 3000 mmol/L, more preferably from 1200 to 2500 mmol/L.

The solution A and the solution B are mixed and allowed to stand for some time to obtain a calcium phosphate polyionic cluster solution. Typically, the solution A and the solution B are mixed in a volume ratio of from 1:1 to 10:1, more preferably from 2:1 to 10:1 and most preferably from 2:1 to 5:1. The standing time is preferably from 10 to 180 minutes, more preferably from 15 to 120 minutes, and most preferably from 20 to 60 minutes.

The calcium phosphate polyionic clusters with fluidity are obtained by separating part of the ethanol solvent through centrifugation of the calcium phosphate polyionic cluster solution. The fluidity of the clusters can be adjusted by the centrifugation speed and time. The higher the centrifugation speed and the longer the centrifugation time, the more viscous the clusters are. Typically, the centrifugation speed is from 5000 to 30000 rpm, more preferably from 6000 to 25000 rpm, and most preferably from 7000 to 22000 rpm. The centrifugation time is preferably from 1 to 80 minutes, more preferably from 1 to 30 minutes and most preferably from 3 to 10 minutes. In a preferred embodiment, the centrifugation speed is from 7000 to 22000 rpm, and the centrifugation time is from 3 to 10 minutes.

Typically, the oral care composition of the present invention comprises from 3 to 80% by weight of the calcium phosphate polyionic clusters, more preferably from 5 to 50%, most preferably from 10 to 50% based on the total weight of the oral care composition and including all ranges subsumed therein.

Glycerin is a component of the present invention. Glycerin typically makes up from 1 to 90%, more preferably from 5 to 80%, and most preferably from 10 to 70% by weight of the oral care composition, based on total weight of the composition and including all ranges subsumed therein.

The oral care composition typically comprises the calcium phosphate polyionic clusters and glycerin in a weight ratio from 1:5 to 10:1, more preferably from 1:5 to 5:1 and most preferably from 1:3 to 2:1.

In one embodiment, the oral care composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition).

For certain compositions, especially anhydrous compositions (i.e. compositions substantially free of water) or dual-phase hydrous compositions, it is preferable to compound a phosphate source in the oral composition.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, tripotassium phosphate, monopotassium dihydrogen phosphate, dipotassium hydrogen phosphate, ammonium phosphate, diammonium hydrogen phosphate, ammonium dihydrogen phosphate, mixtures thereof or the like. The phosphate source is preferably one which is water soluble.

When used, the phosphate source typically makes up from 0.5 to 40%, and more preferably, from 1 to 30%, and most preferably, from 2 to 20% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 mPa.s (centipoise), and preferably, from 60,000 to 170,000 mPa.s (centipoise), and most preferably, from 65,000 to 165,000 mPa.s (centipoise).

The oral care composition of the present invention may include other humectant in addition to glycerin which is included in the composition.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

The oral care composition of this invention can be used in a method for remineralizing teeth of an individual comprising applying the composition to at least one surface of the teeth of an individual. The oral care composition of this invention may additionally or alternatively be for use as a medicament and/or used in the manufacture of a medicament for remineralizing teeth of an individual. Alternatively and preferably, the use is non-therapeutic.

In a preferred embodiment, the oral care composition is a monophase anhydrous composition.

The oral care composition of the present invention is prepared by conventional methods of making oral care compositions. Such methods include mixing the ingredients under moderate shear and atmospheric pressure.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

The following examples are provided to facilitate an understanding of the present invention.

### Examples

### Example 1

This example demonstrated the improved deposition on tooth surfaces. All ingredients are expressed by weight percent of the total formulation, and as level of active ingredient.

**TABLE 1**

| **Ingredient** | **Samples** | | | |
|---|---|---|---|---|
| | **1** | **2** | **3** | **4** |
| Calcium phosphate polyionic cluster | 30 | 30 | 30 | 50 |
| Glycerin | -- | -- | 70 | 50 |
| Ethanol | -- | 70 | -- | -- |
| Water | 70 | -- | -- | -- |

### Preparation of calcium phosphate polyionic clusters

Calcium chloride dihydrate was dissolved in ethanol to obtain a 17 mmol/L calcium chloride solution as solution A. Concentrated phosphoric acid and triethylamine were dissolved in ethanol to give solution B with a phosphoric acid concentration of 40 mmol/L, and a triethylamine concentration of 1300 mmol/L. The solution A and the solution B were mixed in a volume ratio of 4:1 and allowed to stand for 30 minutes to obtain an ethanol solution of calcium phosphate polyionic clusters as solution C. The solution C was centrifuged at 15000 rpm to obtain calcium phosphate polyionic clusters.

### Method

The test sample was mixed with simulated oral fluid (SOF) at a weight ratio of 1:2 for 30 seconds and the resulted slurry was used immediately.

Enamel disks were eroded by 37% phosphoric acid for 15s then they were treated with different slurries following the same protocol. Six enamel disks were separated into three groups (n=2). The enamel disks were soaked in freshly prepared slurry for 2 mins at room temperature then rinsed with 50 mL water for 5s. After that, the enamel disks were soaked in SOF for more than 4 hours under the condition of a water bath at 37°C. The enamel disks were then soaked in freshly prepared slurry using the same procedures as in the first step. The treatment was repeated twice for one day, then the enamel disks were kept in SOF overnight (>12 hours) in a water bath at 37°C to mimic oral environment. The enamel disks were characterized by scanning electron microscopy (SEM, Hitachi S-4800, Japan) after 5 days of twice daily treatments.

Simulated oral fluid was made by combining the ingredients in Table 2:

**TABLE 2**

| **Ingredient** | **Amount/g** |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Results

The eroded enamel disks showed needle-like apatite crystallites. After ten treatments, SEM images of the enamel disks were taken. From the top view of the SEM images, it was apparent that Samples 3 and 4 gave much better and denser deposition on enamel surfaces than Samples 1 and 2. There was no observation of a new remineralization layer formed on enamel surfaces for Samples 1 and 2. The SEM images of the enamel disks treated with Samples 1 or 2 still showed the needle-like apatite crystallites.

The corresponding cross-section SEM images showed that a new layer with a thickness of 1 to 3 microns was formed on the surfaces of the enamel disks after treatment with Samples 3 and 4. The enamel disks treated with Sample 4 had a much thicker new layer formed on their surfaces compared to those treated with Sample 3.

## Claims

1. An oral care composition comprising:
a) calcium phosphate polyionic clusters;
b) glycerin;
wherein the calcium phosphate polyionic clusters are obtained by a process comprising the steps of:
i) dissolving a water soluble calcium source in ethanol to produce an ethanol solution A having a calcium ion concentration from 5 to 60 mmol/L;
ii) dissolving phosphoric acid and triethylamine in ethanol to produce an ethanol solution B having a phosphoric acid concentration from 10 to 120 mmol/L and a triethylamine concentration from 1000 to 3900 mmol/L;
iii) mixing the solution A and the solution B and standing the mixture to obtain a calcium phosphate polyionic cluster solution;
iv) centrifuging the calcium phosphate polyionic cluster solution to obtain the calcium phosphate polyionic clusters
wherein the water soluble calcium source is a calcium source that dissolves in water to give a concentration of at least 0.1 moles per litre at 25°C and atmospheric pressure.

2. The oral care composition according to claim 1, wherein the water soluble calcium source is calcium chloride, calcium nitrate, calcium acetate, calcium lactate, calcium formate, calcium malate, calcium propionate, calcium butyrate, calcium bicarbonate, calcium glycerophosphate, calcium gluconate, calcium ascorbate or mixtures thereof, preferably calcium chloride.

3. The oral care composition according to claim 1 or claim 2, wherein the solution A has a calcium ion concentration from 10 to 50 mmol/L, preferably from 15 to 40 mmol/L.

4. The oral care composition according to any of the preceding claims, wherein the solution B has a phosphoric acid concentration from 20 to 100 mmol/L, preferably from 30 to 80 mmol/L.

5. The oral care composition according to any of the preceding claims, wherein the solution B has a triethylamine concentration from 1100 to 3000 mmol/L, preferably from 1200 to 2500 mmol/L.

6. The oral care composition according to any of the preceding claims, wherein the solution A and the solution B are mixed in a volume ratio of from 1:1 to 10:1, preferably from 2:1 to 10:1, more preferably from 2:1 to 5:1.

7. The oral care composition according to any of the preceding claims, wherein the standing time is from 10 to 180 minutes, preferably from 15 to 120 minutes, and more preferably from 20 to 60 minutes.

8. The oral care composition according to any of the preceding claims, wherein the centrifugation speed is from 5000 to 30000 rpm, preferably from 6000 to 25000 rpm, more preferably from 7000 to 22000 rpm.

9. The oral care composition according to any of the preceding claims, wherein the centrifugation time is from 1 to 80 minutes, preferably from 1 to 30 minutes, more preferably from 3 to 10 minutes.

10. The oral care composition according to any of the preceding claims, wherein the centrifugation speed is from 7000 to 22000 rpm and the centrifugation time is from 3 to 10 minutes.

11. The oral care composition according to any of the preceding claims, wherein the calcium phosphate polyionic clusters are present in an amount from 3 to 80% by weight of the composition, preferably from 5 to 50%.

12. The oral care composition according to any of the preceding claims, wherein the calcium phosphate polyionic clusters and glycerin are present in a weight ratio from 1:5 to 10:1, preferably from 1:5 to 5:1, more preferably from 1:3 to 2:1.

13. The oral care composition according to any of the preceding claims, wherein composition further comprises a phosphate source selected from trisodium phosphate, monosodium dihydrogen phosphate, disodium hydrogen phosphate, tripotassium phosphate, monopotassium dihydrogen phosphate, dipotassium hydrogen phosphate, ammonium phosphate, diammonium hydrogen phosphate, ammonium dihydrogen phosphate or a mixture thereof, preferably trisodium phosphate, monosodium dihydrogen phosphate or mixtures thereof.

14. A composition as defined in any of the preceding claims for use in a method for remineralizing the teeth of an individual comprising the step of applying said composition to at least one surface of the teeth of the individual.

## Patentansprüche

1. Mundpflegezusammensetzung, umfassend:
a) polyionische Calciumphosphatcluster;
b) Glycerin;
wobei die polyionischen Calciumphosphatcluster durch ein Verfahren erhalten werden, das die folgenden Schritte umfasst:
i) Lösen einer wasserlöslichen Calciumquelle in Ethanol, um eine Ethanollösung A mit einer Calciumionenkonzentration von 5 bis 60 mmol/L herzustellen;
ii) Lösen von Phosphorsäure und Triethylamin in Ethanol, um eine Ethanollösung B mit einer Phosphorsäurekonzentration von 10 bis 120 mmol/L und einer Triethylaminkonzentration von 1000 bis 3900 mmol/L herzustellen;
iii) Mischen der Lösung A und der Lösung B und Stehenlassen der Mischung, um eine polyionische Calciumphosphatcluster-Lösung zu erhalten;
iv) Zentrifugieren der polyionischen Calciumphosphatcluster-Lösung, um die polyionischen Calciumphosphatcluster zu erhalten,
wobei die wasserlösliche Calciumquelle eine Calciumquelle ist, die sich in Wasser löst, um eine Konzentration von mindestens 0,1 mol pro Liter bei 25°C und Atmosphärendruck zu ergeben.

2. Mundpflegezusammensetzung nach Anspruch 1, wobei die wasserlösliche Calciumquelle Calciumchlorid, Calciumnitrat, Calciumacetat, Calciumlactat, Calciumformiat, Calciummalat, Calciumpropionat, Calciumbutyrat, Calciumbicarbonat, Calciumglycerophosphat, Calciumgluconat, Calciumascorbat oder Mischungen davon, bevorzugt Calciumchlorid, ist.

3. Mundpflegezusammensetzung nach Anspruch 1 oder Anspruch 2, wobei die Lösung A eine Calciumionenkonzentration von 10 bis 50 mmol/L, bevorzugt von 15 bis 40 mmol/L, aufweist.

4. Mundpflegezusammensetzung nach irgendeinem der vorhergehenden Ansprüche, wobei die Lösung B eine Phosphorsäurekonzentration von 20 bis 100 mmol/L, bevorzugt von 30 bis 80 mmol/L, aufweist.

5. Mundpflegezusammensetzung nach irgendeinem der vorhergehenden Ansprüche, wobei die Lösung B eine Triethylaminkonzentration von 1100 bis 3000 mmol/L, bevorzugt von 1200 bis 2500 mmol/L, aufweist.

6. Mundpflegezusammensetzung nach irgendeinem der vorhergehenden Ansprüche, wobei die Lösung A und die Lösung B in einem Volumenverhältnis von 1:1 bis 10:1, bevorzugt 2:1 bis 10:1, bevorzugter 2:1 bis 5:1, gemischt werden.

7. Mundpflegezusammensetzung nach irgendeinem der vorhergehenden Ansprüche, wobei die Standzeit 10 bis 180 Minuten, bevorzugt 15 bis 120 Minuten und bevorzugter 20 bis 60 Minuten beträgt.

8. Mundpflegezusammensetzung nach irgendeinem der vorhergehenden Ansprüche, wobei die Zentrifugiergeschwindigkeit 5000 bis 30000 U/min, bevorzugt 6000 bis 25000 U/min, bevorzugter 7000 bis 22000 U/min, beträgt.

9. Mundpflegezusammensetzung nach irgendeinem der vorhergehenden Ansprüche, wobei die Zentrifugierdauer 1 bis 80 Minuten, bevorzugt 1 bis 30 Minuten, bevorzugter 3 bis 10 Minuten, beträgt.

10. Mundpflegezusammensetzung nach irgendeinem der vorhergehenden Ansprüche, wobei die Zentrifugiergeschwindigkeit 7000 bis 22000 U/min beträgt und die Zentrifugierdauer 3 bis 10 Minuten beträgt.

11. Mundpflegezusammensetzung nach irgendeinem der vorhergehenden Ansprüche, wobei die polyionischen Calciumphosphatcluster in einer Menge von 3 bis 80 Gewichts-% der Zusammensetzung, bevorzugt von 5 bis 50%, vorhanden sind.

12. Mundpflegezusammensetzung nach irgendeinem der vorhergehenden Ansprüche, wobei die polyionischen Calciumphosphatcluster und Glycerin in einem Gewichtsverhältnis von 1:5 bis 10:1, bevorzugt von 1:5 bis 5:1, bevorzugter von 1:3 bis 2:1, vorhanden sind.

13. Mundpflegezusammensetzung nach irgendeinem der vorhergehenden Ansprüche, wobei die Zusammensetzung ferner eine Phosphatquelle umfasst, die ausgewählt ist aus Trinatriumphosphat, Mononatriumdihydrogenphosphat, Dinatriumhydrogenphosphat, Trikaliumphosphat, Monokaliumdihydrogenphosphat, Dikaliumhydrogenphosphat, Ammoniumphosphat, Diammoniumhydrogenphosphat, Ammoniumdihydrogenphosphat oder einer Mischung davon, bevorzugt Trinatriumphosphat, Mononatriumdihydrogenphosphat oder Mischungen davon.

14. Zusammensetzung wie in irgendeinem der vorhergehenden Ansprüche definiert zur Verwendung in einem Verfahren zur Remineralisierung der Zähne eines Individuums, umfassend den Schritt des Auftragens der Zusammensetzung auf mindestens eine Oberfläche der Zähne des Individuums.

## Revendications

1. Composition pour le soin oral comprenant :
a) des amas polyioniques de phosphate de calcium ;
b) de la glycérine ;
dans laquelle les amas polyioniques de phosphate de calcium sont obtenus par un procédé comprenant les étapes de :
i) dissolution d'une source de calcium soluble dans l'eau dans de l'éthanol pour produire une solution d'éthanol A ayant une concentration en ion calcium de 5 à 60 mmol/L ;
ii) dissolution d'acide phosphorique et triéthylamine dans de l'éthanol pour produire une solution d'éthanol B ayant une concentration en acide phosphorique de 10 à 120 mmol/L et une concentration en triéthylamine de 1 000 à 3 900 mmol/L ;
iii) mélange de la solution A et la solution B et repos du mélange pour obtenir une solution d'amas polyioniques de phosphate de calcium ;
iv) centrifugation de la solution d'amas polyioniques de phosphate de calcium pour obtenir les amas polyioniques de phosphate de calcium
dans laquelle la source de calcium soluble dans l'eau est une source de calcium qui se dissout dans l'eau pour fournir une concentration d'au moins 0,1 mole par litre à 25°C et pression atmosphérique.

2. Composition pour le soin oral selon la revendication 1, dans laquelle la source de calcium soluble dans l'eau est le chlorure de calcium, nitrate de calcium, acétate de calcium, lactate de calcium, formate de calcium, malate de calcium, propionate de calcium, butyrate de calcium, bicarbonate de calcium, glycérophosphate de calcium, gluconate de calcium, ascorbate de calcium ou mélanges de ceux-ci, de préférence chlorure de calcium.

3. Composition pour le soin oral selon la revendication 1 ou revendication 2, dans laquelle la solution A présente une concentration en ion calcium de 10 à 50 mmol/L, de préférence de 15 à 40 mmol/L.

4. Composition pour le soin oral selon l'une quelconque des revendications précédentes, dans laquelle la solution B présente une concentration en acide phosphorique de 20 à 100 mmol/L, de préférence de 30 à 80 mmol/L.

5. Composition pour le soin oral selon l'une quelconque des revendications précédentes, dans laquelle la solution B présente une concentration en triéthylamine de 1 100 à 3 000 mmol/L, de préférence de 1 200 à 2 500 mmol/L.

6. Composition pour le soin oral selon l'une quelconque des revendications précédentes, dans laquelle la solution A et la solution B sont mélangées dans un rapport de volume de 1:1 à 10:1, de préférence de 2:1 à 10:1, encore mieux de 2:1 à 5:1.

7. Composition pour le soin oral selon l'une quelconque des revendications précédentes, dans laquelle la durée de repos est de 10 à 180 minutes, de préférence de 15 à 120 minutes, et encore mieux de 20 à 60 minutes.

8. Composition pour le soin oral selon l'une quelconque des revendications précédentes, dans laquelle la vitesse de centrifugation est de 5 000 à 30 000 tr/min, de préférence de 6 000 à 25 000 tr/min, encore mieux de 7 000 à 22 000 tr/min.

9. Composition pour le soin oral selon l'une quelconque des revendications précédentes, dans laquelle la durée de centrifugation est de 1 à 80 minutes, de préférence de 1 à 30 minutes, encore mieux de 3 à 10 minutes.

10. Composition pour le soin oral selon l'une quelconque des revendications précédentes, dans laquelle la vitesse de centrifugation est de 7 000 à 22 000 tr/min et la durée de centrifugation est de 3 à 10 minutes.

11. Composition pour le soin oral selon l'une quelconque des revendications précédentes, dans laquelle les amas polyioniques de phosphate de calcium sont présents dans une quantité de 3 à 80 % en masse de la composition, de préférence de 5 à 50 %.

12. Composition pour le soin oral selon l'une quelconque des revendications précédentes, dans laquelle les amas polyioniques de phosphate de calcium et la glycérine sont présents dans un rapport massique de 1:5 à 10:1, de préférence de 1:5 à 5:1, encore mieux de 1:3 à 2:1.

13. Composition pour le soin oral selon l'une quelconque des revendications précédentes, dans laquelle la composition comprend de plus une source de phosphate choisie parmi le phosphate de trisodium, dihydrogénophosphate de monosodium, hydrogénophosphate de disodium, phosphate de tripotassium, dihydrogénophosphate de monopotassium, hydrogénophosphate de dipotassium, phosphate d'ammonium, hydrogénophosphate de diammonium, dihydrogénophosphate d'ammonium ou un mélange de ceux-ci, de préférence phosphate de trisodium, dihydrogénophosphate de monosodium ou mélanges de ceux-ci.

14. Composition selon l'une quelconque des revendications précédentes pour une utilisation dans un procédé de reminéralisation des dents d'un individu comprenant l'étape d'application de ladite composition sur au moins une surface des dents de l'individu.
